# EUROPÄISCHE PATENTSCHRIFT

(11) **EP 4 412 680 B1**
(45) Veröffentlichungstag und Bekanntmachung des Hinweises auf die Patenterteilung: **18.11.2026**
(21) Anmeldenummer: 22789594.3
(22) Anmeldetag: 28.09.2022
(51) Int. Cl.: A61M 5/20, A61M 5/32, A61M 5/31, A61M 5/315

(54) **AUTOINJEKTOR MIT ROTIERENDER ANZEIGE**
AUTOINJECTOR WITH ROTATING DISPLAY
INJECTEUR AUTOMATIQUE POURVU D'AFFICHAGE ROTATIF

(30) Priorität: 07.10.2021 EP 21201392
(43) Veröffentlichungstag der Anmeldung: 14.08.2024
(60) Teilanmeldung: 26207901.5
(73) Patentinhaber: Ypsomed AG, 3401 Burgdorf (CH)
(72) Erfinder: HIRSCHEL, Jürg, 3007 Bern (CH); KALBERMATTER, Gabriel, 3400 Burgdorf (CH); GRUENIG, Nicolas, 1784 Courtepin (CH)
(74) Vertreter: Meier Obertüfer, Jürg
(86) Internationale Anmeldenummer: PCT/EP2022/076929
(87) Internationale Veröffentlichungsnummer: WO 2023/057272

(56) Entgegenhaltungen:
- WO-A1-2012/173554
- WO-A1-2016/205963
- US-A1- 2014 330 214

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Anwender zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Anwender, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO 2016/205963 beschreibt einen beispielhaften Autoinjektor, umfassend ein Gehäuse mit Längsachse und einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzfeder gekoppelt ist. Eine Spiral- oder Triebfeder, in welcher Energie für das automatische Ausschütten vom Produkt gespeichert werden kann, ist über ein erstes Ende mit dem Gehäuse und über ein zweites Ende rotationsfest mit einem koaxial zur Längsachse angeordneten Antriebselement in Form einer rotierenden Gewindestange verbunden. Die Gewindestange greift über ein Gewinde in ein im Gehäuse nicht rotierendes Vortriebsglied in Form einer Vortriebshülse, welche bei einer Verschiebung in distale Richtung den Stopfen des Produktbehälters mit einer annähernd konstanten Ausschüttgeschwindigkeit mitbewegt.

Eine Triebfeder als Antrieb zeichnet sich aus durch hohe Kräfte und ist somit geeignet für einen verzögerungsfreien Start der Ausschüttung auch bei Autoinjektoren mit einer langen Lagerdauer. Eine variable Gewindesteigung der Gewindestange kann eine variable Kennlinie der Triebfeder kompensieren um eine möglichst konstante Ausschüttkraft zu gewährleisten. Dadurch können auch grosse Ausschüttmengen von 5 ml oder mehr innerhalb von maximal 60 s gleichmässig und kontinuierlich ausgeschüttet werden. Bei einer gegenüber gängigen Autoinjektoren verlängerten Ausschüttdauer von mehr als 10 s sollte sich der Anwender jederzeit über den bestimmungsgemässen Fortgang der Injektion vergewissern können, um nicht die Injektion in unbeabsichtigter Weise vorzeitig abzubrechen.

Die Patentanmeldung WO 2012/173554 zeigt eine rotierende Anzeige am proximalen Ende eines Autoinjektors, welche durch eine Torsionsfeder während der Ausschüttung angetrieben wird. Die Anzeige umfasst mehrere Segmente mit unterschiedlichen Farben zur Signalisierung eines initialen Zustandes vor Beginn der Ausschüttung und eines finalen Zustandes nach erfolgter Ausschüttung. Die Segmente sind durch mindestens ein Fenster am proximalen Ende des Autoinjektors sichtbar, welches in seiner Ausdehnung dem Winkelbereich eines Segments entspricht.

Die Patentanmeldung US2014/0330214 A1 zeigt eine Anzeige umfassend eine rotierende Scheibe mit mehreren, durch radial verlaufende Rippen getrennten Sektoren, und umfassend eine transparente Kappe mit vier Öffnungen in welchen opake Abdeckungen platziert sind. Durch Letztere wird der Sichtbereich auf die Scheibe unterbrochen beziehungsweise nur in bestimmte Richtungen gewährleistet. Vor und nach der Ausschüttung sind unterschiedlich eingefärbte Sektoren durch die transparenten Abschnitte der Kappe erkennbar, oder Sektoren mit unterschiedlicher Winkelausdehnung, so dass der Nutzer den Zustand des Geräts ableiten kann.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder coformulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter dem Begriff "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist eine Aufgabe der Erfindung, eine Anzeige für einen Autoinjektor der eingangs genannten Art zu schaffen, welche dem Anwender ein Andauern oder Fortschreiten einer Ausschüttung bei unterschiedlichsten Griffpositionen optisch anzeigen kann. Die Anzeige soll so ausgestaltet sein, dass auch bei ungünstigen, insbesondere nicht vorgesehenen Griffpositionen einer Hand des Anwenders am Autoinjektor der Anwender das Andauern der Ausschüttung erkennen kann, ohne dass er dazu die Griffposition wechseln muss. Die Aufgabe wird gelöst durch einen Autoinjektor mit den Merkmalen des unabhängigen Anspruchs 1. Bevorzugte Ausführungsformen der Erfindung sind Gegenstand der abhängigen Ansprüche.

Ein erfindungsgemässer Autoinjektor umfasst ein einteiliges oder mehrteiliges Gehäuse mit einer Längsachse und eine vorgefüllte Fertigspritze mit einem Produktbehälter und einer daran unlösbar befestigten Injektionsnadel oder Kanüle. Die Fertigspritze ist im Gehäuse axial nicht verschiebbar aufgenommen, wobei eine Spitze der Injektionsnadel um mindestens eine Einstechtiefe in distaler Richtung über ein distales Gehäuseende vorsteht. Der Autoinjektor umfasst weiter eine zur einmaligen Ausschüttung eines maximalen Inhalts des Produktbehälters vorgespannte Torsionsfeder, ein Antriebselement, ein Vortriebselement, und eine Nadelschutzhülse. Zur Ausschüttung von Flüssigkeit aus dem Produktbehälter durch die Injektionsnadel versetzt die Torsionsfeder das Antriebselement in Rotation um die Längsachse, und das rotierende Antriebselement bewirkt eine Linearbewegung des Vortriebselements zur Verschiebung eines Kolbens im Produktbehälter. Die Nadelschutzhülse wird beim Anpressen des Autoinjektors an eine Injektionsstelle und dem dadurch bewirkten Einstechen der Injektionsnadel in die Injektionsstelle um einen Betätigungshub in proximale Richtung bewegt und startet oder ermöglicht dadurch eine Ausschüttung von Flüssigkeit. Der Betätigungshub der Nadelschutzhülse entspricht dabei mindestens der Einstechtiefe der Injektionsnadel.

Der Autoinjektor umfasst schliesslich eine Anzeige zur Signalisierung des Andauerns einer Ausschüttung, aufweisend ein durch das Antriebselement angetriebenes, um die Längsachse rotierendes Anzeigeelement mit einem optischen Kontrastmuster, und ein aus einem transparenten und bevorzugt stabilen oder tragfähigen Material gefertigtes Fenster am proximalen Ende des Autoinjektors, durch welches das rotierende Kontrastmuster sichtbar ist. Das Fenster umfasst einen um die Längsachse vollständig um 360° umlaufenden, also nicht durch einen Rahmen oder Steg unterbrochenen Sichtbereich auf das Kontrastmuster. Der allseitig einsehbare Sichtbereich vermeidet blinde Sektoren oder Winkelbereiche, aus welchen die Sicht auf das Kontrastmuster nicht gewährleistet oder unterbrochen ist. Solange ein noch so kleiner Teil des Fensters sichtbar bleibt, also auch wenn der Anwender ungeschickterweise einen überwiegenden Teil des Fensters mit seiner den Autoinjektor haltenden Hand abdeckt, beispielsweise indem er mit dem Handballen am proximalen Ende abstützt, kann das rotierende Anzeigeelement noch erkannt werden.

Das Kontrastmuster ist höchstens diskret rotationssymmetrisch um die Längsachse, aber nicht kontinuierlich rotationssymmetrisch, damit eine Rotation des Kontrastmusters auch erkannt werden kann. Das Kontrastmuster umfasst mindestens eine Linie, eine Fläche oder ein graphisches Element in einem von einer Hintergrundfarbe verschiedenen Farb- oder Grauton oder ausgestaltet als dreidimensionale Oberflächenstruktur des Anzeigeelements. Das Kontrastmuster umfasst bevorzugt sich in Rotationsrichtung wiederholende graphische Elemente, wie beispielsweise parallelen Linien, welche insbesondere zur Längsachse des Autoinjektors geneigt und/oder gekrümmt verlaufen. Das Kontrastmuster kann mindestens eine bis 60, insbesondere zwischen 8 und 16 Linien beziehungsweise Flächen oder grafische Elemente aufweisen. Das Kontrastmuster enthält insbesondere eine der physiologisch gut wahrnehmbaren Farben grün oder schwarz.

Ein Autoinjektor nach der Erfindung ist geeignet zur Ausschüttung der im Produktbehälter enthaltenen Produktmenge während einer gegenüber bekannten Autoinjektoren erhöhten Ausschüttdauer von mehr als 10 s, bevorzugt mehr als 20 s oder 30 s. Dadurch ist auch bei Produktmengen von über 2.25 ml und von bevorzugt mindestens 3 ml oder 4 ml eine kontinuierliche oder durchschnittliche Ausschüttrate relativ gering, so dass die injizierte Menge an Medikament laufend vom subkutanen Gewebe aufgenommen oder resorbiert werden kann. Das Einhalten einer minimalen Haltezeit zwischen Ausschüttende und Wegbewegen des Autoinjektors von der Einstichstelle ist daher weniger kritisch. Entsprechend ist keine optische, akustische, oder taktile Signalisierung vonnöten, welche dem Anwender das Ende einer Haltezeit anzeigt.

In einer bevorzugten Ausführungsform ist das rotierende Anzeigeelement direkt oder unmittelbar, insbesondere ohne Getriebe, durch das Antriebselement angetrieben. Das Anzeigeelement rotiert also mit der derselben Drehgeschwindigkeit wie das Antriebselement.

In einer weiter bevorzugten Ausführungsform führt das Anzeigeelement während der Ausschüttung weniger als eine Umdrehung, bevorzugt weniger als eine halbe Umdrehung oder gar weniger als ein Drittel einer Umdrehung pro Sekunde aus. Dadurch wird sichergestellt, dass die Drehbewegung des Kontrastmusters von Auge gut verfolgbar ist und nicht etwa für den Betrachter zu einem unbestimmten rotationssymmetrischen Muster verwischt.

In einer bevorzugten Ausführungsform ist weder am Fenster noch am anschliessenden Gehäuse einenichtrotierende optische Markierung zur Feststellung einer relativen Drehposition des Kontrastmusters vorgesehen. Durch das Anzeigeelement erfolgt keine Anzeige eines Anfangs- oder Endzustands, auch keine Relativangabe zum Fortschritt der Ausschüttung, es dient ausschliesslich zur Signalisierung des Fortschreitens oder Andauerns der Ausschüttung.

In einer bevorzugten Ausführungsform sind das rotierende Anzeigeelement und das Gehäuse oder das Fenster zur Erzeugung eines Ausschüttgeräusches ausgebildet, wodurch dem Anwender zusätzlich das Andauern des Ausschüttvorganges akustisch signalisiert wird. Bevorzugt ist das Ausschüttgeräusch ein kontinuierliches Klickgeräusch, welches durch ein Raster am Fenster oder am Gehäuse und ein darin eingreifendes radial oder axial flexibles Eingriffselement am Anzeigeelement, oder umgekehrt durch ein rotierendes Raster am Anzeigeelement und ein stationäres Eingriffselement erzeugt wird.

In einer bevorzugten Ausführungsform wird auf eine von der Rotation des Anzeigeelements unabhängige und beispielsweise durch eine Axialbewegung ausgelöste optische, akustische, und/oder taktile Signalisierung des Ausschüttendes verzichtet. Das Erreichen der distalen Endposition des Stopfens wird nicht durch ein dezidiertes mechanisch oder elektronisch erzeugtes Signal angezeigt. Einzig die Tatsache, dass das Anzeigeelement nicht mehr rotiert, zusammen mit der Sicht auf den Stopfen in seiner Endposition durch die als Sichtfenster bezeichneten Ausnehmungen des Gehäuses, bestätigt dem Anwender das Ende der Ausschüttung.

In einer bevorzugten Ausführungsform weist das Fenster einen ersten, zylinderförmigen Bereich parallel zur Längsachse, und einen zweiten, in proximaler Richtung nahtlos an den ersten anschliessenden Bereich mit stetig oder kontinuierlich abnehmendem Durchmesser auf. Der erste Bereich ermöglicht eine Sicht auf das Anzeigeelement aus jeder seitlichen Blickrichtung senkrecht zur Längsachse, und der zweite Bereich ermöglicht eine Sicht auf das Anzeigeelement in distaler Blickrichtung von hinten, beziehungsweise bei senkrecht gehaltenem Autoinjektor von oben. Der zweite Bereich ist in Richtung proximal abgerundet oder einschnürend ausgebildet, und kann in einem dritten Bereich senkrecht zur Längsachse enden. Das Anzeigeelement kann eine dem Verlauf des Fensters folgende konvexe Anzeigefläche aufweisen oder eine kegelmantelförmige Anzeigefläche mit einer mittleren Neigung bezüglich der Längsachse. Bevorzugt ist das Fenster bezüglich der Längsachse rotationssymmetrisch, also mit kreisförmigen Querschnitten in Schnittebenen senkrecht zur Längsachse.

In einer vorteilhaften Variante umfasst der Autoinjektor eine Nadelschutzhülse, welche im Auslieferzustand des Autoinjektors distal über das Gehäuse vorsteht und durch eine Nadelschutzfeder in distale Richtung vorgespannt ist. Bei Anpressen des Autoinjektors an eine Injektionsstelle führt die Nadelschutzhülse eine Betätigungsbewegung in proximale Richtung aus und bei Entfernen des Autoinjektors von der Injektionsstelle eine Nadelschutzbewegung in distaler Richtung, um mit einem hülsenförmigen, bevorzugt rotationssymmetrischen Abschnitt die Injektionsnadel seitlich zu umgeben und radial abzudecken. Die Nadelschutzhülse weist an einem distalen Ende einen ringförmigen Flansch oder Fuss auf als vergrösserte Auflagefläche zum Gewebe um die Injektionsstelle. Der Flansch ist permanent mit dem hülsenförmigen Abschnitt verbunden und bevorzugt einstückig mit diesem ausgebildet. Ein maximaler Durchmesser des Flansches ist grösser als ein maximaler Durchmesser des hülsenförmigen Abschnitts.

Bevorzugt ist der äussere Rand oder die Peripherie des Flansches an die Form einer distalen Öffnung im Gehäuse angepasst. Im eingestochenen Zustand bildet der Flansch somit einen Abschluss des Gehäuses. Weiter bevorzugt ist der Flansch konkav gewölbt ausgebildet, so dass der äussere Rand sich weiter distal befindet als der Übergang zwischen Flansch und hülsenförmigem Abschnitt. Gegenüber einem Flansch mit einer streng ebenen, ringförmigen Auflagefläche mit identischem Aussendurchmesser ist dadurch erstens ein Verkippen des aufgesetzten Autoinjektors weniger wahrscheinlich, und zweitens führt die Konzentration der Druckbelastung auf die von der Einstichstelle am weitesten entfernte Peripherie des Flansches zu weniger Gegendruck im Gewebe und somit zu weniger Schmerz beim Anwender.

Weiter bevorzugt umfasst der Autoinjektor eine Gewindestange als Antriebselement und eine Vortriebshülse mit Innengewinde als Vortriebselement, alternativ eine Antriebshülse mit Innengewinde als Antriebselement und eine Gewindestange als Vortriebselement, wobei das Vortriebselement eine Nut oder einen Nocken als Axialführungselement für eine ausschliesslich lineare Vortriebsbewegung im Gehäuse aufweist. Der Autoinjektor ist bevorzugt dimensioniert zur Aufnahme einer vorgefüllten Fertigspritze umfassend den Produktbehälter und die Injektionsnadel, und aufweisend ein Füllvolumen von mindestens 3 ml, bevorzugt mindestens 5 ml.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig.1: die Komponenten eines Autoinjektors,
- Fig.2: zwei Längsschnitte durch den Autoinjektor aus Fig.1 vor der Injektion,
- Fig.3: zwei Ansichten des Anzeigeelements;
- Fig.4: eine Ansicht des distalen Endes der Nadelschutzhülse, und
- Fig.5: eine Ansicht eines Anzeigeelements mit radial elastischem Ausschüttklicker.

### FIGURENBESCHREIBUNG

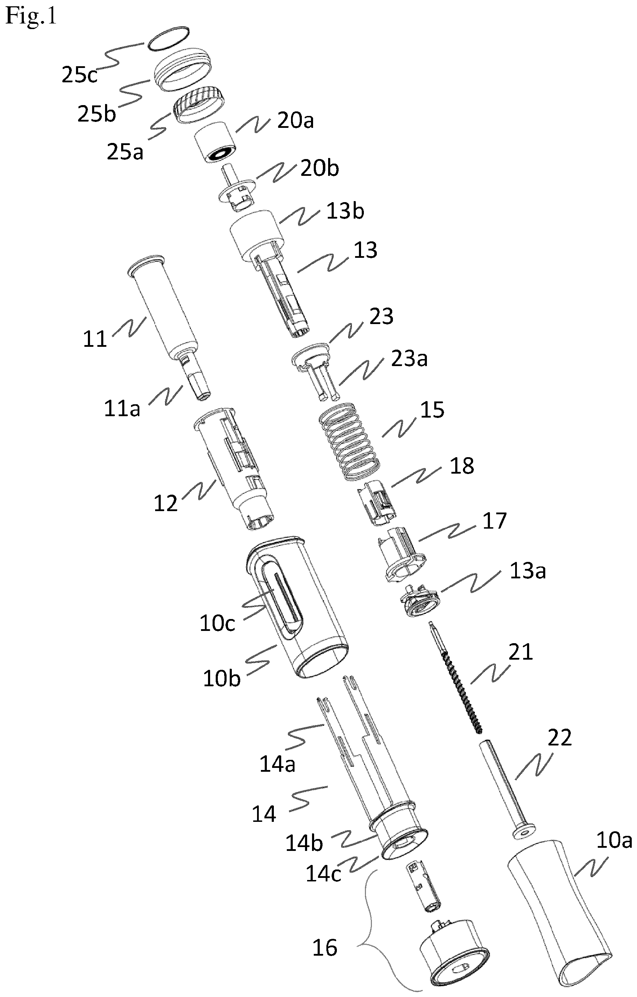

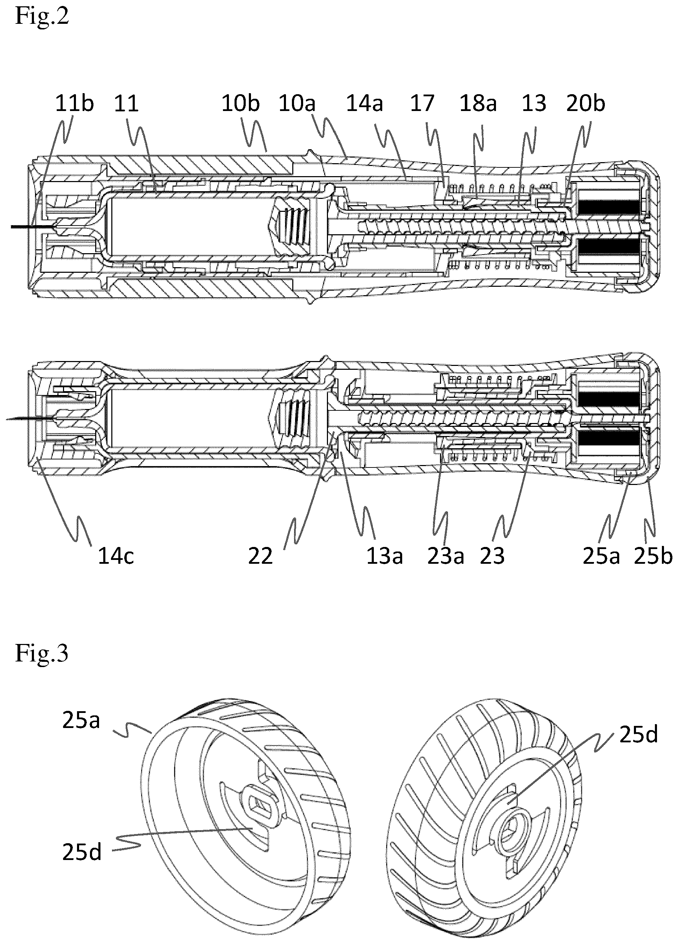

Fig.1 ist eine Explosionsdarstellung der Komponenten eines Autoinjektors nach der Erfindung, und Fig.2 zeigt zwei zueinander um die Längsachse um 90° gedrehte Längsschnitte durch den Autoinjektor nach Fig.1 im eingestochenen Zustand und bereit zur Ausschüttung.

Der Autoinjektor weist ein hülsenförmiges, längliches Gehäuse auf mit einer Längsachse L und umfassend ein distales Gehäuseteil 10b und ein damit unlösbar verschnapptes proximales Gehäuseteil 10a in Form eines Griffs. Ein Produktbehälter in Form einer Fertigspritze 11 mit einer am Produktbehälter unlösbar befestigten Injektionsnadel ist in einem Spritzenhalter 12 gehalten, wobei der Spritzenhalter im Gehäuse axial- und drehfest aufgenommen ist. Die Fertigspritze 11 wird von einem fest im Gehäuseteil 10a verankerten Haltefederabschnitt 13a in distale Richtung in einen Eingriff mit einer Schulter des Spritzenhalters 12 gedrückt. Die Fertigspritze 11 ist in Bezug auf das Gehäuseteil 10b so angeordnet, dass die Spitze der Injektionsnadel um eine der subkutanen oder intramuskulären Einstechtiefe entsprechende Länge über das distale Ende einer proximalen Zwischenposition der Nadelschutzhülse 14 hinausragt und durch eine Nadelschutzhülse 14 vor und nach der Injektion zumindest seitlich geschützt oder abgedeckt wird. Die Nadelschutzhülse 14 wird beim Einstechen der Injektionsnadel in die Injektionsstelle entlang der Längsachse L um einen Betätigungshub und gegen die Kraft einer Nadelschutzfeder 15 in proximale Richtung geschoben und löst dadurch eine Produktausschüttung aus. Die Nadelschutzhülse umfasst dazu zwei Hülsenarme 14a, welche gegenüber zwei als Sichtfenster bezeichneten Ausnehmungen 10c des Gehäuses um 90° um die Längsachse L versetzt beziehungsweise verdreht angeordnet sind. Nach der erfolgten Injektion kann die Nadelschutzhülse 14 relativ zu dem Gehäuseteil 10b aus der betätigten Position entlang der Längsachse L in die distale Richtung in eine Nadelschutzposition verschoben und dort gegen erneutes Zurückschieben blockiert werden. Die Nadelschutzhülse umfasst einen hülsenförmigen oder hohlzylindrischen Abschnitt 14b und einen Flansch 14c am distalen Ende. Der Querschnitt des hohlzylindrischen Abschnitts 14b ist oval, genauso wie der äussere Umfang des Flansches, so dass vorliegend der Flansch eine konstante Breite oder radiale Ausdehnung aufweist.

Ein Federpaket umfasst eine Spiralfeder 20a und eine Federspule 20b. Die Spiralfeder 20a ist mit ihrem äusseren Ende drehfest an einer Federhülse 13b als Teil eines fest im Gehäuse verankerten Mechanikhalters 13 befestigt. Das innere Ende der Spiralfeder 20a ist drehfest mit der Federspule 20b verbunden. Die Federspule 20b umfasst einen Federschaft und einen distalen Federflansch. Die Spiralfeder 20a beziehungsweise die Federspule 20b versetzt ein Antriebselement 21 in eine Rotationsbewegung und ein Vortriebselement 22 in eine bevorzugt rein axiale Vortriebsbewegung. Dazu greift ein Gewindeelement in ein sich über den Ausschütthub erstreckendes Gewinde mit einer variablen Gewindesteigung.

Die Fertigspritze 11 umfasst einen zylindrischen Spritzenkörper als Produktbehälter, an dessen distalen Ende eine hohle Injektionsnadel mit einer Spritzenschulter fest verbunden ist. Die Injektionsnadel der Fertigspritze ist von einer Nadelschutzkappe 11a abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe schützt die Injektionsnadel gegen mechanische Einwirkungen und Verschmutzung, und hält die Injektionsnadel und das Produkt steril. An dem distalen Ende des Autoinjektors ist in seinem Auslieferungszustand eine zweiteilige Geräte- oder Abziehkappe 16 angeordnet, die vor der Verwendung des Autoinjektors zusammen mit der Nadelschutzkappe 11a axial abgezogen und/oder abgedreht und vollständig entfernt wird.

Eine Schalthülse 17 ist formschlüssig mit einem proximalen Ende der Hülsenarme 14a der Nadelschutzhülse 14 und mit einem distalen Ende der Nadelschutzfeder 15 angeordnet und zumindest teilweise von Letzterer umgeben. Die Schalthülse 17 ist bevorzugt mit dem proximalen Ende der Hülsenarme der Nadelschutzhülse 14 verschnappt. Die Bewegung der Schalthülse 17 in distale Richtung ist durch den Haltefederabschnitt 13a begrenzt, welcher seinerseits nach der Montage der Schalthülse 17 mit dem Mechanikhalter 13 verschnappt. Innerhalb und koaxial zur Schalthülse 17 ist eine Sperrhülse 18 angeordnet, welche über ein sägezahnförmiges, an einem in distale Richtung weisenden Arm federnd angebrachtes Verriegelungsglied 18a derart an die Schalthülse 17 gekoppelt ist, dass eine Betätigungsbewegung von Nadelschutzhülse 14 und Schalthülse 17 auch die Sperrhülse 18 nach proximal bewegt. Durch einen zusätzlichen proximalen Arretierhub der Sperrhülse 18 relativ zur Schalthülse 17 in eine proximale Endposition wird das Verriegelungsglied 18a von der Schalthülse 17 für eine Bewegung nach innen sicher freigegeben. Durch die Federwirkung des Arms hintergreift das Verriegelungsglied 18a eine nach proximal gerichtete Kante des Autoinjektors oder rastet in eine axialfeste Ausnehmung des Autoinjektors ein und arretiert so die Sperrhülse 18 gegen eine distale Bewegung. Beim Entfernen des Autoinjektors von der Einstichstelle wird die Schalthülse 17 durch die Nadelschutzfeder 15 in distaler Richtung über das Verriegelungsglied 18a geschoben, worauf das Verriegelungsglied durch die Federwirkung des Arms in einer Verriegelungsposition eine nach proximal gerichtete Kante der Schalthülse 17 hintergreift und die Schalthülse sowie die Nadelschutzhülse gegen eine erneute Bewegung in proximale Richtung verriegelt oder blockiert.

Eine Kupplungshülse 23 mit zwei Haltenocken 23a ist über Kopplungselemente mit der Federspule 20b gekoppelt. Vor der Ausschüttung greifen die Haltenocken 23a in Ausnehmungen des axialfesten Mechanikhalters 13 ein und sind durch einen Innenumfang der Sperrhülse 18 an einer Bewegung nach aussen gehindert, wodurch sich die Kupplungshülse 23 auch axial nicht bewegen kann. Beim Auslösen der Ausschüttung wird die Sperrhülse 18 durch eine proximale Bewegung der Nadelschutzhülse 14 von der Position der Ausnehmungen wegbewegt, so dass sich die Haltenocken 23a radial lösen können und die Kupplungshülse 23 freigeben wird. Letztere bewegt sich in proximaler Richtung und gibt die Federspule 20b zur Rotation frei, wie ausführlich beschrieben in der Patentanmeldung PCTEP2021076923. Am proximalen Ende des Autoinjektors befindet sich die Anzeige mit einem rotierenden Anzeigeelement 25a mit einem Kontrastmuster in Form von parallelen Streifen, einem umlaufenden transparenten Fenster 25b, und einem proximalen, nicht-transparenten Abschluss 25c.

Fig.3 zeigt zwei Ansichten des Anzeigeelements 25a mit einem Eingriffselement 25d zum Eingriff in ein nichtrotierendes Raster an der Innenseite des Fensters 25b oder des Abschlusses 25d. Eingriffselement 25d ist federnd gelagert und erzeugt bei Rotation des Anzeigeelements während der Ausschüttung eine der Rasterung entsprechende Anzahl von Klickgeräuschen. Das Anzeigeelement hat eine Form welche derjenigen des Fensters folgt beziehungsweise an diese angepasst ist, mit einem ersten, distalen Bereich und einem in proximaler Richtung daran anschliessenden zweiten Bereich. Der erste Bereich ist zylinderförmig, parallel zur Längsachse, während der zweite Bereich einen stetig abnehmenden Durchmesser aufweist.

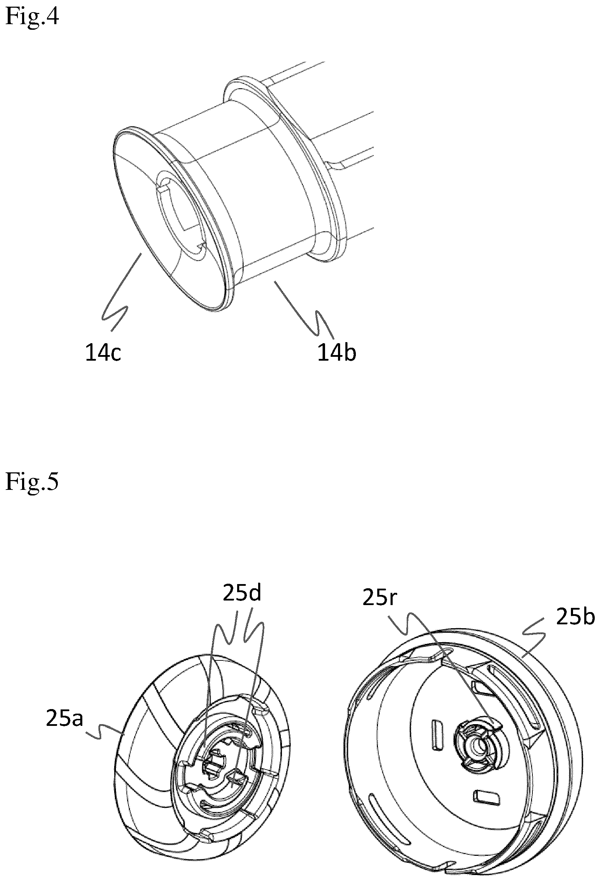

Fig.4 zeigt das distale Ende der Nadelschutzhülse mit dem hülsenartigen Abschnitt 14b und dem Flansch 14c. Deutlich zu sehen ist die Form des Flansches mit der konkav gewölbten Auflagefläche, deren äusserer, ovaler Rand gegenüber dem inneren Rand der Auflagefläche beim Übergang zum achsenparallelen Abschnitt nach distal vorsteht.

Fig.5 zeigt eine Ansicht einer zweiten Ausführungsform des Anzeigeelements 25a und des Fensters 25b. Das Anzeigeelement weist zwei radial elastisch oder federnd ausgebildete Eingriffselemente 25d auf, welche über Rastnocken am freien Ende mit vier um 90° versetzten Rasterungen 25r auf der Innenseite des Fensters zusammenwirken. Bei Rotation des Anzeigeelementes im Gegenuhrzeigersinn (in distale Richtung betrachtet) werden die Nocken durch spiralförmig verlaufende Leitflächen der Gegenelemente nach aussen gelenkt und schnappen am Ende der Leitflächen wieder radial nach innen. Dadurch wird ein Klickgeräusch erzeugt, und durch die Form des Eingriffs gleichzeitig eine Rotation des Anzeigeelements im Uhrzeigersinn verhindert. Anders als bei der axialen Federung des Eingriffselementes aus Fig.3 ist hier die Federvorspannung und das Klickverhalten unabhängig von der exakten axialen Position des Anzeigeelements, eine gewisse Toleranz oder Spiel diesbezüglich ist also nicht kritisch. Der kleine Reibradius der radial auslenkenden Eingriffselemente führt auch zu geringen Reibverlusten und damit zu einem minimalen Einfluss auf das Ausschüttverhalten.

Das vorliegend beschriebene Kontrastmuster kann im Tampondruckverfahren oder durch Thermotransferdruck auf den Grundkörper des Anzeigeelements aufgedruckt werden. Ebenso kann das Kontrastmuster mittels 2-Komponenten Spritzguss in unterschiedlicher Farbe hergestellt werden. Bevorzugt werden dabei die beiden Komponenten aufgeteilt, wobei die erste Komponente, vorzugsweise aus PBT die funktionellen Elemente, wie beispielsweise die Schnapper beinhaltet. Dadurch werden die funktionalen Elemente immer mit demselben Material und vorzugsweise in gleicher Farbe erzeugt. Die visuellen, ästhetischen Elemente wie das Kontrastmuster können mit der zweiten Komponente, vorzugsweise aus eingefärbtem ABS oder PP erzeugt werden und farblich variieren.

Das Kontrastmuster kann auch mittels Laserverfahren, vorzugsweise mittels UV-Laser erzeugt werden, indem am Anzeigeelement auf der Sichtfläche lokal Material abgetragen und dadurch eine veränderte insbesondere graue Struktur erzeugt wird. Auch kann durch ein Laserverfahren und geeignete Materialwahl die Oberfläche des Anzeigeelements angeschmolzen werden, wodurch Pigmente sichtbar werden oder der Kunststoff aufgrund seiner Inhaltsstoffe aufschäumt und dadurch die optischen Eigenschaften kontrastierend verändert werden. Alternativ kann mittels Laser oder Erosion auf der zuvor polierten Sichtfläche des Spritzgusswerkzeugs lokal und feinstrukturiert Material abgetragen werden, wodurch Strukturen, insbesondere Dach- oder Würfelstrukturen erzeugt werden können, welche später beim Spritzgiessen auf das Kunststoffteil übertragen werden. Solche Oberflächenstrukturen können eine Tiefe bzw. Erhabenheit von 0,01 bis 0,5 mm aufweisen. Diese ist aus Gründen der Nachhaltigkeit eine vorteilhafte Ausführung, da für das Bauteil weder unterschiedliche Materialien gemischt, noch Farbe auf das Material aufgetragen oder das Material lokal durch eine Laserbeschriftung verändert wird.

Das Kontrastmuster kann auch mittels Durchbrüchen in der Sichtfläche des Anzeigeelements geschaffen werden. Dabei erlauben die Durchbrüche den Blick in das dunkel erscheinende Innere des Autoinjektors, insbesondere auf die metallische spiralförmige Torsionsfeder.

**BEZUGSZEICHENLISTE**

| | | | |
|---|---|---|---|
| 10a, 10b | Gehäuseteil | 16 | Gerätekappe |
| 10c | Ausnehmung | 17 | Schalthülse |
| 11 | Fertigspritze | 18 | Sperrhülse |
| 11a | Nadelschutzkappe | 18a | Verriegelungsglied |
| 11b | Injektionsnadel | 20a | Spiralfeder |
| 12 | Spritzenhalter | 20b | Federspule |
| 13 | Mechanikhalter | 21 | Antriebselement |
| 13a | Haltefederabschnitt | 22 | Vortriebselement |
| 13b | Federhülse | 23 | Kupplungshülse |
| 14 | Nadelschutzhülse | 23a | Haltenocken |
| 14a | Arm | 25a | Anzeigeelement |
| 14b | Abschnitt | 25b | Fenster |
| 14c | Flansch | 25c | Abschluss |
| 15 | Nadelschutzfeder | 25d | Eingriffselement |

## Patentansprüche

1. Autoinjektor umfassend
- ein Gehäuse (10a, 10b) mit einer Längsachse L und zur Aufnahme eines Produktbehälters (11),
- ein Vortriebsglied (22) und ein rotierendes Antriebselement (21) zur Bewegung des Vortriebsglieds (22) in Längsrichtung und zur automatischen Ausschüttung eines in dem Produktbehälter (11) enthaltenen flüssigen Produkts durch eine Injektionsnadel (11b) am distalen Ende des Autoinjektors,
- eine Anzeige zur Signalisierung des Andauerns einer Ausschüttung, umfassend ein durch das Antriebselement (21) angetriebenes rotierendes Anzeigeelement (25a) mit einem optischen Kontrastmuster, und umfassend ein Fenster (25b) am proximalen Ende des Autoinjektors, durch welches das rotierende Kontrastmuster sichtbar ist, wobei das Fenster (25b) ein um die Längsachse vollständig umlaufendes, ununterbrochenes Fenster ist, **dadurch gekennzeichnet, dass** das Fenster (25b) einen um 360° umlaufenden, allseitig einsehbaren, nicht unterbrochenen Sichtbereich auf das Kontrastmuster umfasst.

2. Autoinjektor nach Anspruch 1, **dadurch gekennzeichnet, dass** das rotierende Anzeigeelement (25a) direkt durch das Antriebselement (21) angetrieben ist.

3. Autoinjektor nach Anspruch 1, **dadurch gekennzeichnet, dass** keine optische Markierung zur Feststellung einer Drehposition des Kontrastmusters vorgesehen ist.

4. Autoinjektor nach Anspruch 1, **dadurch gekennzeichnet, dass** das rotierende Anzeigeelement (25a) zur Erzeugung eines Ausschüttgeräusches ausgebildet ist, wobei das Anzeigeelement (25a) zur Erzeugung von Klickgeräuschen ein flexibles Eingriffselement (25d) aufweist, welches in ein Raster (25r) am Fenster (25b) oder am Gehäuse eingreift.

5. Autoinjektor nach einem der vorhergehenden Ansprüche, **dadurch gekennzeichnet, dass** keine unabhängige optische, akustische, und/oder taktile Signalisierung des Ausschüttendes vorgesehen ist.

6. Autoinjektor nach einem der vorhergehenden Ansprüche, **dadurch gekennzeichnet, dass** das Fenster (25b) einen ersten, zylinderförmigen Bereich parallel zur Längsachse, und einen zweiten, in proximaler Richtung nahtlos an den ersten anschliessenden Bereich mit stetig abnehmendem Durchmesser aufweist.

7. Autoinjektor nach einem der vorhergehenden Ansprüche, weiter umfassend - eine Nadelschutzhülse (14), welche im Auslieferzustand des Autoinjektors distal über das Gehäuse vorsteht, wobei bei Anpressen des Autoinjektors an eine Injektionsstelle die Nadelschutzhülse (14) eine Betätigungsbewegung in proximale Richtung ausführt und bei Entfernen des Autoinjektors von der Injektionsstelle eine Nadelschutzbewegung in distaler Richtung ausführt, um mit einem hülsenförmigen Abschnitt (14b) die Injektionsnadel (11b) seitlich zu umgeben, **dadurch gekennzeichnet, dass** die Nadelschutzhülse (14) an einem distalen Ende einen ringförmigen Flansch (14c) zum Kontakt mit der Injektionsstelle aufweist, mit einem maximalen Durchmesser, welcher grösser ist als der maximale Durchmesser des hülsenförmigen Abschnitts (14b).

8. Autoinjektor nach Anspruch 7, **dadurch gekennzeichnet, dass** ein äusserer Rand des Flanschs (14c) an eine distale Öffnung im Gehäuse (10b) angepasst ist.

9. Autoinjektor nach Anspruch 7, **dadurch gekennzeichnet, dass** der Flansch (14c) konkav gewölbt ausgebildet ist.

10. Autoinjektor nach einem der vorhergehenden Ansprüche, **dadurch gekennzeichnet, dass** das Antriebselement (21) eine Gewindestange ist und dass das Vortriebselement (22) eine Vortriebshülse mit einem Axialführungselement für eine ausschliesslich lineare Vortriebsbewegung im Gehäuse (10b) ist.

11. Autoinjektor nach einem der vorhergehenden Ansprüche, **gekennzeichnet durch** eine vorgefüllte Fertigspritze umfassend den Produktbehälter (11) und die Injektionsnadel (11b), und aufweisend ein Füllvolumen von mindestens 3 ml, bevorzugt mindestens 5 ml.

## Claims

1. Autoinjector comprising
- a housing (10a, 10b) having a longitudinal axis L and for accommodating a product container (11),
- a propulsion member (22) and a rotating drive element (21) for moving the propulsion member (22) in the longitudinal direction and for automatically discharging a liquid product contained in the product container (11) through an injection needle (11b) at the distal end of the autoinjector,
- a display for signaling the persistence of a discharge, comprising a rotating display element (25a) which is driven by the drive element (21) and has an optical contrast pattern, and comprising a window (25b) at the proximal end of the autoinjector, through which window the rotating contrast pattern is visible, the window (25b) being an uninterrupted window which fully extends about the longitudinal axis, **characterized in that** the window (25b) comprises an uninterrupted field of vision onto the contrast pattern that extends by 360° and can be viewed from all sides.

2. Autoinjector according to claim 1, **characterized in that** the rotating display element (25a) is driven directly by the drive element (21).

3. Autoinjector according to claim 1, **characterized in that** no optical marking is provided for determining a rotational position of the contrast pattern.

4. Autoinjector according to claim 1, **characterized in that** the rotating display element (25a) is designed to generate a discharging noise, the display element (25a) having a flexible engagement element (25d) for generating clicking noises that engages in a grid (25r) on the window (25b) or on the housing.

5. Autoinjector according to any of the preceding claims,
**characterized in that** no independent optical, acoustic, and/or tactile signaling of the end of the discharge is provided.

6. Autoinjector according to any of the preceding claims,
**characterized in that** the window (25b) has a first, cylindrical region parallel to the longitudinal axis, and a second region which seamlessly adjoins the first in the proximal direction and has a constantly decreasing diameter.

7. Autoinjector according to any of the preceding claims, further comprising - a needle guard sleeve (14) which, in the delivered state of the autoinjector, projects distally beyond the housing, the needle guard sleeve (14) performing an actuating movement in a proximal direction when the autoinjector is pressed against an injection site and performing a needle guard movement in a distal direction when the autoinjector is removed from the injection site in order to laterally surround the injection needle (11b) using a sleeve-shaped portion (14b), **characterized in that** the needle protection sleeve (14) has, at a distal end, an annular flange (14c) for contact with the injection site, having a maximum diameter which is larger than the maximum diameter of the sleeve-shaped portion (14b).

8. Autoinjector according to claim 7, **characterized in that** an outer edge of the flange (14c) is adapted to a distal opening in the housing (10b).

9. Autoinjector according to claim 7, **characterized in that** the flange (14c) is designed to be concavely curved.

10. Autoinjector according to any of the preceding claims,
**characterized in that** the drive element (21) is a threaded rod and **in that** the propulsion element (22) is a propulsion sleeve having an axial guide element for an exclusively linear propulsion movement in the housing (10b).

11. Autoinjector according to any of the preceding claims, **characterized by** a pre-filled, ready-to-use syringe comprising the product container (11) and the injection needle (11b), and having a filling volume of at least 3 ml, preferably at least 5 ml.

## Revendications

1. Auto-injecteur comprenant
- un boîtier (10a, 10b) comportant un axe longitudinal L et destiné à recevoir un contenant de produit (11),
- un organe d'avancement (22) et un élément d'entraînement (21) rotatif destiné à déplacer l'organe d'avancement (22) dans le sens longitudinal et à distribuer automatiquement un produit liquide contenu dans le contenant de produit (11) grâce à une aiguille d'injection (11b) située à l'extrémité distale de l'auto-injecteur,
- un indicateur destiné à signaler la durée d'une distribution, comprenant un élément indicateur (25a) rotatif entraîné par l'élément d'entraînement (21) et comportant un motif de contraste optique, et comprenant une fenêtre (25b) à l'extrémité proximale de l'auto-injecteur, à travers laquelle le motif de contraste rotatif est visible, dans lequel la fenêtre (25b) est une fenêtre ininterrompue s'étendant sur toute la circonférence autour de l'axe longitudinal, **caractérisé en ce que** la fenêtre (25b) comprend un champ visuel ininterrompu sur le motif de contraste, lequel champ visuel s'étend sur 360° et est visible de tous les côtés.

2. Auto-injecteur selon la revendication 1, **caractérisé en ce que** l'élément indicateur (25a) rotatif est entraîné directement par l'élément d'entraînement (21).

3. Auto-injecteur selon la revendication 1, **caractérisé en ce qu'**aucun marquage optique permettant de déterminer une position de rotation du motif de contraste n'est prévu.

4. Auto-injecteur selon la revendication 1, **caractérisé en ce que** l'élément indicateur (25a) rotatif est conçu pour produire un bruit de distribution, dans lequel l'élément indicateur (25a) présente, pour la production de bruits de cliquetis, un élément de mise en prise (25d) flexible qui vient en prise dans une grille (25r) sur la fenêtre (25b) ou sur le boîtier.

5. Auto-injecteur selon l'une des revendications précédentes,
**caractérisé en ce qu'**aucune signalisation optique, sonore et/ou tactile indépendante de l'extrémité de distribution n'est prévue.

6. Auto-injecteur selon l'une des revendications précédentes,
**caractérisé en ce que** la fenêtre (25b) présente une première zone cylindrique parallèle à l'axe longitudinal et une seconde zone, se raccordant sans discontinuité à la première zone dans le sens proximal, le diamètre de la seconde zone diminuant progressivement.

7. Auto-injecteur selon l'une des revendications précédentes, comprenant en outre : - un manchon de protection d'aiguille (14) qui, à l'état de l'auto-injecteur à la livraison, fait saillie distalement au-delà du boîtier, dans lequel le manchon de protection d'aiguille (14), lorsque l'auto-injecteur est pressé contre un site d'injection, effectue un déplacement d'actionnement dans le sens proximal et, lors du retrait de l'auto-injecteur du site d'injection, effectue un déplacement de protection d'aiguille dans le sens distal afin d'entourer latéralement l'aiguille d'injection (11b) avec une section (14b) en forme de manchon, **caractérisé en ce que** le manchon de protection d'aiguille (14) présente, à une extrémité distale, une bride annulaire (14c) destinée à entrer en contact avec le site d'injection, bride annulaire dont le diamètre maximal est supérieur au diamètre maximal de la section (14b) en forme de manchon.

8. Auto-injecteur selon la revendication 7, **caractérisé en ce qu'**un bord extérieur de la bride (14c) est adapté à une ouverture distale dans le boîtier (10b).

9. Auto-injecteur selon la revendication 7, **caractérisé en ce que** la bride (14c) est réalisée avec une courbure concave.

10. Auto-injecteur selon l'une des revendications précédentes,
**caractérisé en ce que** l'élément d'entraînement (21) est une tige filetée et **en ce que** l'élément d'avancement (22) est un manchon d'avancement comportant un élément de guidage axial permettant un déplacement d'avancement exclusivement linéaire dans le boîtier (10b).

11. Auto-injecteur selon l'une des revendications précédentes,
**caractérisé par** une seringue préremplie comprenant le contenant de produit (11) et l'aiguille d'injection (11b), et présentant un volume de remplissage d'au moins 3 ml, de préférence d'au moins 5 ml.
